# EUROPEAN PATENT SPECIFICATION

(11) **EP 1 454 142 B1**
(45) Date of publication and mention of the grant of the patent: **21.12.2011**
(21) Application number: 02787885.9
(22) Date of filing: 30.11.2002
(51) Int. Cl.: G01N 33/68, C12Q 1/68

(54) **INHIBITION OF TRISTETRAPROLINE FOR PROTECTION OF THE HEART FROM CARDIAC INJURIES**
INHIBITION VON TRISTETRAPROLIN ZUM SCHUTZ VON HERZERKRANKUNGEN
INHIBITION DE LA TRISTETRAPROLINE VISANT A PROTEGER LE COEUR CONTRE LES LESIONS CARDIAQUES

(30) Priority: 12.12.2001 EP 01129564
(43) Date of publication of application: 08.09.2004
(73) Proprietor: Merck Patent GmbH, 64293 Darmstadt (DE)
(72) Inventor: KLUXEN, Franz-Werner, 64367 Mühltal (DE); HENTSCH, Bernd, 60322 Frankfurt (DE); EHRING, Thomas, 42853 Remscheid (DE); BRÄNDLE, Marian, 72181 Starzach (DE); HOHEISEL, Jörg, Dietrich, 69168 Wiesloch (DE); FROHME, Marcus, 68535 Edingen (DE); ZUBAKOV, Dimitri, 69117 Heidelberg (DE)
(86) International application number: PCT/EP2002/013545
(87) International publication number: WO 2003/054541

(56) References cited:
- WO-A-01/12213
- WO-A-97/42820
- US-B1- 6 187 543
- P. HWU ET AL.: "Functional and molecular characterization of tumor-infiltrating lymphocytes transduced with tumor necrosis factor alpha cDNA for gene therapy of cancer", JOURNAL OF IMMUNOLOGY, vol. 150, 1993, pages 4104-4115, Bethesda MD USA

## Description

### Field of the invention

The present invention relates to compounds suitable for the treatment of conditions which may be improved, at least in part, by increasing tissue necrosis factor α (TNFα) production. The invention further relates to assays for the identification of an individual at increased risk for or suffering from such conditions and to methods of screening compounds for their ability to enhance TNFα biosynthesis.

### Background of the invention

TNFα is a potent cytokine that is released from many cell types. TNFα also exists in a cell-membrane bound, higher molecular weight form on cells, and this form also appears to mediate a variety of biological effects. TNFα is thought to have few roles in normal development and physiology; however, it exerts harmful and destructive effects on many tissues in many disease states (Tracey et al., Ann. Rev. Med 1994; 45:491). Disease states in which TNFα has been shown to exert a major role include septic shock syndrome, cancer cachexia, rheumatoid arthritis, etc.

In contrast, experimental studies from several laboratories have shown that the adult mammalian heart synthesizes TNFα mRNA after certain forms of stress (Bader et al., Proc. Natl. Acad. Sci. USA. 1994; 91:11831; Giroir et al., J. Clin. Invest. 1992; 90:693; Giroir et al., Am. J. Physiol. 1994; 267:H118.)

The repeated observation that TNFα is expressed in virtually all forms of cardiac injury in both large and small mammals, including but not limited to myocardial infarction (Mauri et al., J. Intern. Med. 1989; 225:333; Basaran et al., Angiology. 1993; 44:332), unstable angina (Matsumori et al., Br. Heart J. 1994; 72:566), hemodynamic overloading (Kapadia et al., J. Clin. Invest. 1995; 96:1042), myocardial reperfusion injury (Lefer et al., Science. 1990; 249:61; Herskowitz et al., Am. J. Pathol. 1995;146:419), hypertrophic cardiomyopathy (Matsumori et al., Br. Heart. J. 1994;72:561), and end-stage congestive heart failure (Levine et al., N. Engl. J. Med. 1990; 223:236; Torre-Amione et al., Circulation. 1996; 93:704) suggests that TNFα may act as a phylogenetically conserved "innate stress response gene" in the heart.

In this regard, the observation that TNFα confers resistance to hypoxic stress in the adult cardiac tissue suggests that TNFα production by the injured and/or stressed cardiac tissue may serve as a local autocrine/paracrine/ juxtacrine mechanism for protecting neighboring cells within the heart (Nakano et al., Circulation. 1998; 97:1392)

It is well known that short periods of coronary artery occlusion followed by reperfusion confers protection of the heart from later periods of otherwise lethal ischemic conditions. However, this first protective period ends after several hours. 24h after the initial ischemia, a 'second window of protection' (SWOP) occurs, which may last for several days, depending on the species used and the experimental conditions. Besides occlusion/reperfusion periods, a number of substances like adenosine and lipopolysaccharide (LPS) are also known to induce a SWOP.

Among others, the expression of iNOS (inducible NO-synthase) is considered to be an important mediator of the SWOP. iNOS as itself is induced by TNFα, which is expressed e.g. after induction of a SWOP by LPS. The expression of TNFα seems to be necessary for the development of the SWOP. Therefore an elevated level of TNFα may thus be of therapeutic use in conditions which may be improved, at least in part, by increasing TNFα production including but not limited to cardiac diseases and/or cardiac injuries and/or for the prevention of reperfusion damage.

The present invention relates to a novel approach to the treatment of conditions which may be improved, at least in part, by increasing TNFα production. This approach involves the use of a cDNA or a cRNA complementary to the TTP mRNA or an anti-TTP antibody specifically binding to TTP or fragments of said antibodies.

TTP (Lai et al, J. Biol. Chem. 1990; 265:16556), also known as Nup475 (DuBois et al, J. Biol. Chem. 1990; 265:19185) and TIS11 (Varnum et al, Oncogene 1989; 4:119; Varnum et al, Mol. Cell. Biol. 1991; 11:1754), is a widely distributed 33 kDa phosphoprotein encoded by the immediate-early response gene, Zfp-36 (Taylor et al, Nucl. Acids Res. 1991; 19:3454). This gene has been mapped to chromosome 7 in the mouse, and the equivalent human gene, ZFP36, has been mapped to chromosome 19q 13.1 (Taylor et al, Nucl. Acids Res. 1991; 19:3454). TTP is the prototype of a group of proteins containing two or more highly conserved putative zinc fingers of the CCCH class (Varnum et al, Mol. Cell. Biol. 1991; 11:1754; Taylor et al, Nucleic Acids Res. 1991; 19:3454; Gomperts et al, Oncogene. 1990; 5:1081; Ma et al, Oncogene. 1994; 9:3329). In addition, the protein has been shown to bind Zn²⁺ and has been localized to the cell nucleus in mouse fibroblasts (DuBois et al, J. Biol. Chem. 1990; 265:19185), suggesting that it may be a transcription factor. Serum or other mitogen stimulation of quiescent fibroblasts causes rapid serine phosphorylation and nuclear to cytosolic translocation of TTP (Taylor et al., J. Biol. Chem. 1995; 270:13341), both of which are likely to modulate its function in cells.

Recently it was shown that TTP plays a role in TNFα regulation in macrophages (Carballo et al., Science 1998; 281:2001). TTP expression is induced by lippoysaccharides and TNFα. TTP is able to bind to an AU-rich element (ARE) in the 3 prime end of the TNFα mRNA molecule, thus destabilizing the TNFα mRNA.

In WO 97/42820 the properties of TTP have been used for the treatment of diseases which are associated with TNFα excess like septic shock syndrome, cancer achexia, rheumatoid arthritis etc. Methods of treatment of such diseases by increasing the TTP level are described as well as methods for selecting compounds for their ability to inhibit TNFα production and methods for identifying an individual at increased risk to the effects of TNFα excess.

WO 01/12213 provides methods of regulating the destruction of mRNA molecules containing an AU-rich element (ARE), for example, methods of stimulating the degradation of an mRNA molecule encoding TNFα and methods of inhibiting the degradation of an mRNA molecule encoding granulocyte-macrophage stimulating factor for the treatment of granulocytopenia.

The function of TTP in an individual at risk for or suffering from a condition which may be improved, at least in part, by increasing TNFα production including but not limited to cardiac diseases and/or cardiac injuries was heretofore unknown.

### Summary of the invention

It is an object of the present invention to provide screening assays for selecting compounds for their ability to increase TNFα production.

A further object of the present invention is the use of a cDNA or a cRNA complementary to the TTP mRNA, said cDNA or cRNA binding to and inhibiting the translation of the TTP mRNA or an anti-TTP antibody specifically binding to TTP and inhibiting the binding of TTP to TNFα mRNA or fragments of said antibodies selected from the group consisting of F(ab')₂-, F(ab)₂-, Fab'-, Fab-, Fv-fragments, scFv proteins, 3.5S Fab' monovalent fragments and CDR peptides for the manufacture of a medicament for the treatment of cardiac diseases and/or cardiac injuries selected from the group consisting of hemodynamic overloading, myocardial reperfusion injury, hypertrophic cardiomyopathy, end-stage congestive heart failure, myocardial infarction and unstable angina and medicaments comprising these compounds.

It is also an embodiment of the present invention to provide the use of the active compounds for the manufacture of a medicament for the treatment of an individual at risk for or suffering from conditions which may be improved, at least in part, by increasing TNFα production including but not limited to cardiac diseases and/or cardiac injuries and/or for the prevention of reperfusion damage.

Further objects and advantages of the invention will be clear from the description that follows.

### Brief description of the figures

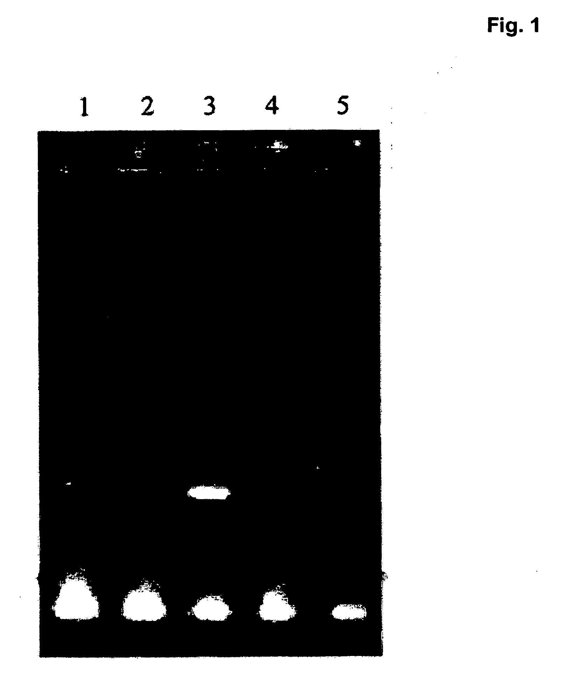

**Figure 1**. Detection of TTP mRNA in dog hearts by RT-PCR.
1. 8h reperfusion control (sham-surgery)
2. 4h reperfusion control (sham-surgery)
3. 16h reperfusion (surgery)
4. 8h reperfusion (surgery)
5. 4h reperfusion (surgery)
   RNAs from the indicated sources were used for RT-PCR analysis. RNAs were reverse trancribed using standard methods and subsequently subjected to PCR using short oligodesoxynukleotides as primers designed from the original dog TTP sequence.

### Detailed description of the invention

The objects of the present invention are achieved on the basis of the unexpected finding that the expression of the protein tristetraproline (TTP) is upregulated in a time dependent manner in cardiac tissue after occlusion of a coronary artery and following reperfusion (Fig. 1).

As indicated above, TNFα production by the injured and/or stressed cardiac tissue may serve as a local autocrine/paracrine/ juxtacrine mechanism for protecting neighboring cells. Accordingly, the ability of TTP to regulate TNFα production in heart is of considerable pharmaceutical importance.

The finding that TTP is expressed in heart after occlusion of a coronary artery followed by reperfusion and the availability of the amino acid sequence of human TTP (Taylor et al, Nucl. Acids Res. 1991, 19:3454) and its encoding sequence (eg GenBank accession number M63625) permits the development of assays for screening compounds which can be used for the prevention and/or treatment of conditions which may be improved, at least in part, by increasing TNFα production including but not limited to cardiac diseases, cardiac injuries and/or for the prevention of reperfusion damage and of methods for identifying and diagnosing individuals at increased risk for or suffering from such conditions.

The term "cardiac diseases and/or cardiac injuries" refers to diseases and/or injuries like or hemodynamic overloading, myocardial reperfusion injury, hypertrophic cardiomyopathy, end-stage congestive heart failure and/or a ischemic condition or a consequence thereof like myocardial infarction or unstable angina.

In one example of a screening assay according to the invention, the full length human TTP cDNA is inserted in a suitable eukaryotic expression vector (under control of a strong promoter like SV40, CMV, or an inducible promoter (e.g. tet on-off system)) carrying a selection marker like neomycin, zeozin, hygromycin, or other substances for selection of recombinant cells. This expression vector is transfected by standard methods into a cell line like H9C2, COS-1, COS-7, HEK293, HEK293 EBNA, CHO, BHK HeLA or similar cell lines for the expression of cDNAs and selected for a stable cell line by adding the antibiotic. In this way, either a monoclonal or a polyclonal cell line is selected.

A second plasmid is constructed using a promoter like SV40, CMV or similar fused to TNFα gene or a reporter gene like luciferase, CAT, ß-glactosidase and a part of the 3' untranslated region of the TNFα mRNA which encompasses the ARE (AU rich element) fragments known to destabilize the TNFα mRNA (bases 1197 to 1350 of GenBank accession number X02611). The vector in addition carries a selection marker that is different from the first vector. Transfection can be effected using art-recognized techniques.

After transfection and selection of the second plasmid the cell line can be used for detecting substances that interfere with the activity of TTP.

Alternatively, the plasmid carrying TTP and the plasmid carrying the TNFα gene or reporter gene can be mixed and subsequently transfected using standard techniques in a cell line as described above.

Such a cell line can be used for an assay for the screening of a compound for its ability to enhance TNFα production by inhibiting and/or reverse the binding of TTP to TNFα mRNA comprising for instance the steps:
a) contacting the compound with the cell line expressing TTP and TNFα or, instead of TNFα, a part of the 3'untranslated region of the TNFα mRNA which encompasses the ARE fragments known to destabilize the TNFα mRNA fused to a repoter gene, and
b) determining the level of expression of TNFα or of the reporter protein and comparing that level to a level of expression obtained in the absence of the compound, and
c) selecting compounds which enhance the expression of TNFα or the expression of the reporter protein in comparison to that level of expression obtained in the absence of the compound.

The test compounds are added and after 24 to 72 h the activity of the reporter gene is measured using an enzyme activity reader or a fluorescence signal detector, or in the case wherein the cell line expresses TNFα, with antibodies directed against TNFα or other art-recognized techniques; the observed values are used for the determination of the activity of TTP.

Substances that increase TTP activity can be detected by a higher activity of the reporter gene than in comparison to the non-treated control cells.

A substance that inhibits and/or reverses the binding of TTP to the ARE located in the 3' end of the TNFα or reporter mRNA will stabilize the mRNA and lead to a higher production of TNFα or reporter protein than in the absence of such a compound.

In another example of a screening assay according to the invention the assay comprises the following steps:
a) contacting the compound to be screened with a cell-free sample comprising an expression construct able to express a part of the 3' untranslated region of the TNF-α mRNA which encompasses the ARE fragments known to destabilize the TNF-α mRNA fused to a reporter gene in the presence of TTP under conditions such that the reporter protein can be expressed, and
b) determining the level of expression of the reporter protein and comparing that level to a level of expression obtained in the absence of the compound, and
c) selecting compounds which enhance the expression of the reporter protein in comparison to that level of expression obtained in the absence of the compound.

A further example of a screening assay according to the invention the assay comprises the following steps:
a) contacting said compound with a cell-free sample comprising TNFα mRNA, in the presence of TTP under conditions such that expression of TNFα can be expressed, and
b) determining the level of expression of TNFα and comparing the level of produced TNFα to a level of TNFα obtained in the absence of said compound and
c) selecting compounds which enhance the expression of the reporter protein in comparison to that level of expression obtained in the absence of the compound.

Compounds which inhibit and/or reverse the inhibitory activity of TTP can then be further assayed, using standard protocols, for stability, toxicity etc.

In one approach for identifying and/or diagnosing individuals at increased risk for or suffering from conditions which may be improved, at least in part, by increasing TNFα production including but not limited to cardiac diseases and/or cardiac injuries or for identifying individuals needing a therapy for the prevention of reperfusion damage, a biological sample obtainable from said individuals may be examined for the presence of elevated amounts of TTP or TTP-mRNA.

Fore example, the detection of TTP can be effected by contacting the sample with an anti-TTP antibody and measuring the amount of TTP.

These assays can be accomplished by any of a number of methods. Such methods include immunoassays which include but are not limited to competitive and non-competitive assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement fixation assays, immunoradiometric assays, fluorescent immunoassays, protein A immunoassays, to name but a few.

For example such an assay can be carried out by a method comprising the following steps:
a) contacting a biological sample obtainable from said individual with anti-TTP antibodies under conditions that binding of the antibody to TTP can occur
b) measuring the amount of TTP by ELISA or a method as mentioned above.

The anti-TTP antibody can be detected by a second antibody which further comprises a detectable label. The detectable label may be a radioisotope that is detected by autoradiography. Isotopes that are particularly useful for the purpose of the present invention are ³H, ¹²⁵I, ¹³¹I, ³⁵S and ¹⁴C. The second antibodies may also be labeled with a fluorescent compound. The presence of a fluorescently-labeled antibody is determined by exposing the immunoconjugate to light of the proper wavelength and detecting the resultant fluorescence. Fluorescent labeling compounds include fluorescein, isothiocyanate, rhodamine, phycoerytherin, phycocyanin, allophycocyanin, o-phthaldehyde and fluorescamine.

Alternatively, the second antibody can be detectably labeled by coupling it to a chemiluminescent compound. The presence of the chemiluminescent-tagged immunoconjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester.

Similarly, a bioluminescent compound can be used to label the second antibody. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and acquorin.

Alternatively, the second antibody can be detectably labeled by linking the second antibody to an enzyme. When the antibody-enzyme conjugate is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include ß-galactosidase, glucose oxidase, peroxidase and alkaline phosphatase.

Those of skill in the art will know of other suitable labels which can be employed in accordance with the present invention. The binding of marker moieties to antibodies can be accomplished using standard techniques known to the art. Typical methodology in this regard is described by Kennedy et al., Clin. Chim. Acta 1976, 70:1; Schurs et al., Clin. Chim. Acta 1977, 81:1; Shih et al., Intl J. Cancer 1990, 46: 1101; Stein et al., Cancer Res. 1990, 50:1330.

TTP or fragments thereof used for the production of antibodies can be synthesized recombinantly using common expression systems such as E. coli, baculovirus, Cos cells, Sf9 cells or eukaryotic cells as described in standard literature (e.g. Ausubel et al.(eds.): Current Protocols in Molecular Biology. John Wiley & Sons Inc., New York). The cDNA may also be modified by a tag like His(6), GST, FLAG or similar to aid the purification of the protein. The protein/polypeptide can then be purified by standard methods including chromatography (e.g., ion exchange, affinity, and size exclusion column chromatography), centrifugation, differential solubility, electrophoresis, or by any standard technique for purification of proteins.

Alternatively, TTP can be isolated from natural sources, using art recognized techniques as described above.

Polyclonal antibodies to recombinant TTP or to TTP isolated from natural sources used for the assay can be prepared using methods well-known to those of skill in the art. (See, for example, Green et al., "Production of Polyclonal Antisera," in Immunochemical Protocols, Manson, ed., pages 1-5, Humana Press 1992; Williams et al., In DNA Cloning 2: Expression Systems, 2nd Edition, Glover et al. (eds.), page 15, Oxford University Press 1995).

The immunogenicity of TTP can be increased through the use of an adjuvant, such as alum (aluminum hydroxide) or Freund's complete or incomplete adjuvant. Polypeptides useful for immunization also include fusion polypeptides, such as fusions of TTP or a portion thereof with an immunoglobulin polypeptide or with maltose binding protein. The polypeptide immunogen may be a full-length molecule or a portion thereof. If the polypeptide portion is "hapten-like," such portion may be advantageously joined or linked to a macromolecular carrier (such as keyhole limpet hemocyanin (KLH), bovine serum albumin (BSA) or tetanus toxoid) for immunization.

Although polyclonal antibodies are typically raised in animals such as horses, cows, dogs, chicken, rats, mice, rabbits, guinea pigs, goats, or sheep, an anti-Zsig62 antibody of the present invention may also be derived from a subhuman primate antibody. General techniques for raising diagnostically and therapeutically useful antibodies in baboons may be found, for example, in WO 91/11465, and in Losman et al., Int. J. Cancer. 1990, 46: 310.

Alternatively, monoclonal anti-TTP antibodies can be generated. Rodent monoclonal antibodies to specific antigens may be obtained by methods known to those skilled in the art (see, for example, Kohler et al., Nature 1975, 256: 495; Coligan et al. (eds.), Current Protocols in Immunology, Vol. 1, pages 2.5.1- 2.6.7 (John Wiley & Sons 1991), Picksley et al., "Production of monoclonal antibodies against proteins expressed in E coli," in DNA Cloning 2: Expression Systems, 2nd Edition, Glover et al. (eds.), page 93 (Oxford University Press 1995)). Briefly, monoclonal antibodies can be obtained by injecting mice with a composition comprising TTP, verifying the presence of antibody production by removing a serum sample, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures. For example an anti-TTP antibody of the present invention may be derived from a human monoclonal antibody. Human monoclonal antibodies are obtained from transgenic mice that have been engineered to produce specific human antibodies in response to antigenic, challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas.

Methods for obtaining human antibodies from transgenic mice are described, for example, by Green et al., Nature Genet. 1994, 7: 13; Lonberg et al., Nature 1994, 368:856; Taylor et al., Int. Immun. 1994, 6: 579.

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example Baines et al., "Purification of Immunoglobulin G (IgG)," in Methods in Molecular Biology, Vol. 10, pages 79-104 (The Humana Press, Inc. 1992)).

In an alternative approach for identifying and diagnosing individuals at increased risk for or suffering from conditions which may be improved, at least in part, by increasing TNFα production including but not limited to cardiac diseases and/or cardiac injuries or for identifying individuals needing a therapy for the prevention of reperfusion damage, a biological sample obtainable from said individual may be examined for the presence of elevated amounts of TTP-mRNA.

For example, the detection of TTP mRNA can be effected as follows:
a) contacting a RNA-containing biological sample obtained from said individual with a detectable cDNA or cRNA of TTP or a fragment thereof able to bind mRNA of TTP under conditions that hybridization with mRNA of TTP can occur
b) measuring the amount of TTP-mRNA by any hybridization based method like northern blot, dot blot, RNAse protection or similar methods relying on the hybridization of TTP cDNA or cRNA to the target RNA derived from the samples to be analyzed.

In a further approach of the invention TTP cDNA derived sequences may be used for the design of primers that can be applied for any PCR based method for the quantitation of TTP mRNA. The quantitation of TTP mRNA levels in biological samples may be used as a diagnostic tool to detect altered gene expression.

According to the present invention the term "cDNA" or "cRNA" refers to a single-stranded DNA or RNA molecule that is formed from an mRNA template by the enzyme reverse transcriptase. Typically, a primer complementary to portions of mRNA is employed for the initiation of reverse transcription.

The above conditions are meant to serve as a guide and it is well within the abilities of one skilled in the art to adapt these conditions for use with a particular assay.

The finding that the expression of TTP is upregulated in cardiac tissue after occlusion of a coronary artery and following reperfusion provides a basis for therapy especially for the treatment of cardiac diseases and/or cardiac injuries and/or for a therapy for the prevention of reperfusion damage

For example, the individual may be treated with anti-TTP antibodies, preferably humanized antibodies or antibody fragments directed to TTP to inhibit the binding of TTP to TNFα mRNA and therefore to increase the production of TNFα.

The term "antibody fragment" in the meaning of the present invention refers to a portion of an antibody such as F(ab')₂, F(ab)₂, Fab', Fab, and the like. Regardless of structure, an antibody fragment binds with the same antigen that is recognized by the intact antibody. The term also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

The term "humanized antibodies" refers to recombinant proteins in which murine complementarity determining regions of a monoclonal antibody have been transferred from heavy and light variable chains of the murine immunoglobulin into a human variable domain.

Such antibodies can be produced as described above. For particular uses, it may be desirable to prepare fragments of anti-TTP antibodies. Such antibody fragments can be obtained, for example, by proteolytic hydrolysis of the antibody. Antibody fragments can be obtained by pepsin or papain digestion of whole antibodies by conventional methods. As an illustration, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, in U.S. patent No. 4,331,647, Nisonoff et al., Arch Biochem. Biophys. 1960, 89:230; Porter, Biochem. J. 1959, 73:119; Edelman et al., in Methods in Enzymology Vol. 1, page 422 (Academic Press 1967). Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

For example, Fv fragments comprise an association of V_{H} and V_{L} chains. This association can be noncovalent, as described by Inbar et al. , Proc. Natl Acad. Sci. USA 1972, 69: 2659. Alternatively, the variable chains can be linked by an intermolecular disulfide bond or cross-linked by chemicals such as glutaraldehyde (see, for example, Sandhu, Crit. Rev. Biotech. 1992, 12: 437).

The Fv fragments may comprise V_{H} and V_{L} chains which are connected by a peptide linker. These single-chain antigen binding proteins (scFv) are prepared by constructing a structural gene comprising DNA sequences encoding the V_{H} and V_{L} domains which are connected by an oligonucleotide. The structural gene is inserted into an expression vector which is subsequently introduced into a host cell, such as E. coli. The recombinant host cells synthesize a single polypeptide chain with a linker peptide bridging the two V domains. Methods for producing scFvs are described, for example, Bird et al., Science 1988, 242: 423; U.S.-Patent No. 4,946,778; Pack et al., Biol.Technology 1993, 11: 1271.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody- producing cells (see, for example, Larrick et al., Methods: A Companion to Methods in Enzymology 2:106 (1991), Courtenay-Luck, "Genetic Manipulation of Monoclonal Antibodies," in Monoclonal Antibodies: Production, Engineering and Clinical Application, Ritter et al. (eds.), page 166, Cambridge University Press 1995, and Ward et al., "Genetic Manipulation and Expression of Antibodies," in Monoclonal Antibodies: Principles and Applications, Birch et al., (eds.), page 137, Wiley-Liss, Inc. 1995).

Alternatively, an anti-TTP antibody may be derived from a humanized monoclonal antibody. Humanized monoclonal antibodies are produced by transferring mouse complementary determining regions from heavy and light variable chains of the mouse immunoglobulin into a human variable domain. Typical residues of human antibodies are then substituted in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi et al., Proc. Natl Acad Sci. USA 1989, 86: 3833. Techniques for producing humanized monoclonal antibodies are described, for example, by Jones et al., Nature 1986, 321: 522; Carter et al., Proc. Natl. Acad. Sci. USA 1992, 89: 4285; Sandhu, Crit. Rev. Biotech. 1992, 12: 437; Singer et al.., J. Immun. 1993, 150: 2844 and U.S. Patent No. 5,693,762.

An alternative therapy regimens may be the use of cDNA or cRNA sequences of TTP or portions thereof. Protective end groups and naturally occurring modifications to the RNA or DNA moieties provide further stability allowing the molecules to be effective over several days. Such modified DNA or RNA moieties are commercially available from ATUGEN, Berlin, Germany.

Such modified or unmodified cDNA or cRNA may be used for the hybridization with TTP mRNA and therefore for the suppression of TTP production.

Delivery of the nucleic acid to cells can be via a variety of mechanisms. The nucleic acid can be present in combination with any agent which aids in the introduction of the DNA into cells. Various sterile solutions may be used for administration of the composition, including water, PBS, etc. The concentration of the DNA will be sufficient to provide a therapeutic dose.

Actual delivery of the cDNA, cRNA, antibodies or fragments thereof, formulated as described above, can be carried out by a variety of techniques including direct injection, administration to the lung and other epithelial surfaces, intravenous injection and other physical methods.

An example for a suitable transfer method according to the present invention is the physical transfer of cDNA or cRNA in liposomes or lipid mixtures using commercially available liposome preparations or lipid mixtures such as Lipofectine, Lipofectaminee (GIBCO-BRL, Inc., Gaithersburg, MD), Superfecto (Qiagen, Inc. Hilden, Germany) and Transfectarno (Promega Biotec, Inc., Madison, WI), as well as other liposomes developed according to procedures standard in the art.directly into target cells. Once in the cell, the cDNA or cRNA complementary to the TTP mRNA binds to TTP mRNA and inhibits translation of TTP which results in a increase of TNFα production. Such lipid mixtures are also commercially available from ATUGEN, Berlin, Germany. Liposome-mediated DNA transfer has been described by various investigators (Liu et al, Gene Therapy 1994, 1:7; Miller and Vile, FASEB J. 1995, 9:190).

The exact method of introducing the nucleic acid into mammalian cells is, of course, not limited to the method described above. Other techniques are widely available for this procedure including the use such as receptor-mediated and other endocytosis mechanism. This invention can be used in conjunction with any of these or other commonly used transfer methods.

The nucleic acids and nucleic acid delivery vehicles of this invention, can be in a pharmaceutically acceptable carrier for in vivo administration to a subject. By "pharmaceutically acceptable" is meant a material that is not biologically or otherwise undesirable, i.e., the material may be administered to a subject, along with the nucleic acid or vehicle, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The nucleic acid or vehicle may be administered orally, parenterally (e.g. , intravenously), by intramuscular injection, by intraperitoneal injection, transdermally, extracorporeally, topically or the like. The exact amount of the nucleic acid or vector required will vary from subject to subject, depending on the species, age, weight and general condition of the subject, the severity or mechanism of any disorder being treated, the particular nucleic acid or vehicle used, its mode of administration and the like.

The compounds selected by an assay as described above, anti-TTP antibodies and fragments thereof can be administered orally, parenterally (e.g., intravenously), by intramuscular injection, by intraperitoneal injection, subcutaneous injection, transdermally, extracorporeally, topically, mucosally or the like.

Depending on the intended mode of administration, the compounds of the present invention can be in pharmaceutical compositions in the form of solid, semi- solid or liquid dosage forms, such as, for example, tablets, suppositories, pills, capsules, powders, liquids, suspensions, lotions, creams, gels, or the like, preferably in unit dosage form suitable for single administration of a precise dosage. The compositions will include, as noted above, an effective amount of the selected composition, possibly in combination with a pharmaceutically acceptable carrier and, in addition, may include other medicinal agents, pharmaceutical agents, carriers, adjuvants, diluents, etc. Parenteral administration of the compounds of the present invention, if used, is generally characterized by injection. Injectables can be prepared in conventional forms, either as liquid solutions or suspensions, solid forms suitable for solution of suspension in liquid prior to injection, or as emulsions. As used herein, "parenteral administration" includes intradermal, subcutaneous, intramuscular, intraperitoneal, intravenous and intratracheal routes. A more recently revised approach for parenteral administration involves use of a slow release or sustained release system such that a constant dosage is maintained (see, e.g., U.S. Patent No. 3,610,795). These compounds can be present in a pharmaceutically acceptable carrier, which can also include a suitable adjuvant. By "pharmaceutically acceptable" is meant a material that is not biologically or otherwise undesirable, i.e., the material may be administered to an individual along with the selected compound without causing substantial deleterious biological effects or interacting in a deleterious manner with any of the other components of the composition in which it is contained.

Unit doses according to the invention may contain daily required amounts of the compound according to the invention, or sub-multiples thereof to make up the desired dose. The optimum therapeutically acceptable dosage and dose rate for a given patient (mammals, including humans) depends on a variety of factors, such as the activity of the specific active compound employed, the age, body weight, general health, sex, diet, time and route of administration, rate of clearance, the object of the treatment, i. e., therapy or prophylaxis and the nature of the disease to be treated which are known to the skilled person.

Therefore, in compositions and combinations in a treated patient (in vivo) a pharmaceutical effective daily dose of the active compound of this invention is between about 0.01 and 100 mg/kg body weight, preferably between 0.1 and 10 mg/kg body weight. According to the application form one single dose may contain between 0.01 and 10 mg of the active compound.

### Example

In anesthetized, open-chest dogs, the second window of protection of ischemic preconditioning was investigated. The dogs were instrumented for measurement of left ventricular pressure and aortic pressure. The dogs were subjected to 5 min of coronary occlusion of the left anterior descending coronary artery perfusing the anterior wall followed either by 4hrs, 8hrs or 16 hrs of reperfusion. A second set of dogs (control group) were not subjected to coronary occlusion. Following the experimental protocol, transmural blocks of myocardial tissue from the center of the anterior wall and the posterior control wall were taken and immediately frozen. To identify mRNAs that are upregulated in dog hearts that underwent occlusion followed by reperfusion for different time periods, RNAs from these hearts were subjected to RDA (representational difference analysis). The method is designed to identify mRNAs that are present in higher amounts in the first, often the treated, tissue (designated as 'tester') than in the second tissue (designated as driver). RNA from shock-frozen left ventricular myocardium was extracted by using a standard guanidinium thiocyanate extraction, followed by centrifugation in a cesium chloride gradient (Pharmacia, LKB, Freiburg, Germany). Poly(A)-RNA was isolated by using oligo-d(T) coupled to magnetic beads (DYNABEADS, Dynal, Norway) according to the manufacturer's instructions. To overcome individual alterations in gene expression mRNA from three non-failing control hearts (Driver) and three failing hearts (Tester) were compared as well as a pooled sample. After reverse transcription (cDNA synthesis system, Gibco) double-stranded cDNAs were digested with DpnII, ligated to linkers, amplified, and subtracted as described for RDA (Hubank, M. and Schatz, D.G. (1994): Nucleic Acids Res.,22, 5640-5648) with minor modifications (Frohme et al.(2000): Ann N Y Acad Sci., 910, 85-104). Three rounds of subtractive hybridization were performed resulting in difference product 2 and 3 which were cloned into pBluescript II-SK+ in DH5alpha E.coli. Inserts of the clone libraries were amplified by standard PCR for gel analysis. Sequencing followed standard procedures for the automated sequencer (Applied Biosystems, USA). BLASTX and BLASTN analyses were used to screen for DNA and protein homologies of the sequences identified.

## Claims

1. A method for screening a compound for its ability to enhance tissue necrosis factor α (TNFα) production by inhibiting and/or reversing the binding of tristetraproline (TTP) to TNFα mRNA comprising:
a) contacting the compound with a cell-free sample comprising TNFα mRNA or with a cell-free sample comprising an expression construct able to express a part of the 3' untranslated region of the TNF-α mRNA which encompasses the AU-rich element (ARE) fragments known to destabilize the TNF-α mRNA fused to a reporter gene in the presence of TTP under conditions such that the reporter protein can be expressed, and
b) determining the level of expression of TNFα or of the reporter protein and comparing that level to a level of expression obtained in the absence of the compound, and
c) selecting compounds which enhance the expression of the reporter protein in comparison to that level of expression obtained in the absence of the compound.

2. A method for screening a compound for its ability to enhance TNFα production by inhibiting and/or reversing the binding of TTP to TNFα mRNA comprising:
a) contacting the compound with a cell line expressing TTP and TNFα or, instead of TNFα, a part of the 3' untranslated region of the TNFα mRNA which encompasses the ARE fragments known to destabilize the TNF-α mRNA fused to a reporter gene, and
b) determining the level of expression of TNFα or of the reporter protein and comparing that level to a level of expression obtained in the absence of the compound, and
c) selecting compounds which enhance the expression of TNFα or the expression of the reporter protein in comparison to that level of expression obtained in the absence of the compound.

3. Use of a cDNA or a cRNA complementary to the TTP mRNA, said cDNA or cRNA binding to and inhibiting the translation of the TTP mRNA or an anti-TTP antibody specifically binding to TTP and inhibiting the binding of TTP to TNFα mRNA or fragments of said antibodies selected from the group consisting of F(ab')₂-, F(ab)₂-, Fab'-, Fab-, Fv-fragments, scFv proteins, 3.5S Fab' monovalent fragments and CDR peptides for the manufacture of a medicament for the treatment of cardiac diseases and/or cardiac injuries selected from the group consisting of hemodynamic overloading, myocardial reperfusion injury, hypertrophic cardiomyopathy, end-stage congestive heart failure, myocardial infarction and unstable angina.

4. A medicament for the treatment of cardiac diseases and/or cardiac injuries selected from the group consisting of hemodynamic overloading, myocardial reperfusion injury, hypertrophic cardiomyopathy, end-stage congestive heart failure, myocardial infarction and unstable angina, comprising at least a cDNA or a cRNA complementary to the TTP mRNA, said cDNA or cRNA binding to and inhibiting the translation of the TTP mRNA or an anti-TTP antibody specifically binding to TTP and inhibiting the binding of TTP to TNFα mRNA or fragments of said antibodies selected from the group consisting of F(ab')₂-, F(ab)₂-, Fab'-, Fab-, Fv-fragments, scFv proteins, 3.5S Fab' monovalent fragments and CDR peptides and optionally a pharmaceutically acceptable carrier, diluent or excipient.

## Patentansprüche

1. Verfahren zum Screening einer Verbindung auf ihre Fähigkeit, die Produktion von Gewebenekrosefaktor-α (TNFα) durch Hemmung und/oder Rückgängigmachen der Bindung von Tristetraprolin (TTP) an TNFα-mRNA zu verstärken, umfassend:
a) das Zusammenbringen der Verbindung mit einer zellfreien Probe, die TNFα-mRNA enthält, oder mit einer zellfreien Probe, die ein Expressionskonstrukt enthält, das in der Lage ist, einen Teil der 3'-untranslatierten Region der TNFα-mRNA, der die AU-reiches-Element- (ARE-) Fragmente beinhaltet, von denen bekannt ist, dass sie TNFα-mRNA destabilisieren, fusioniert an ein Reportergen, in Gegenwart von TTP unter derartigen Bedingungen zu exprimieren, dass das Reporterprotein exprimiert werden kann, und
b) das Bestimmen des Spiegels der Expression von TNFα oder des Reporterproteins und Vergleichen dieses Spiegels mit einem Expressionsspiegel, der in Abwesenheit der Verbindung erhalten wird, und
c) dasa Auswählen von Verbindungen, die die Expression des Reporterproteins im Vergleich zu dem Expressionsspiegel, der in Abwesenheit der Verbindung erhalten wird, verstärken.

2. Verfahren zum Screening einer Verbindung auf ihre Fähigkeit, die TNFα-Produktion durch Hemmung und/oder Rückgängigmachen der Bindung von TTP an TNFα-mRNA zu verstärken, umfassend:
a) das Zusammenbringen der Verbindung mit einer Zelllinie, die TTP und TNFα oder, anstelle von TNFα, einen Teil der 3'-untranslatierten Region der TNFα-mRNA, die die ARE-Fragmente beinhaltet, von denen bekannt ist, dass sie die TNFα-mRNA destabilisieren, fusioniert an ein Reportergen, exprimiert, und
b) das Bestimmen des Spiegels der Expression von TNFα oder des Reporterproteins und Vergleichen dieses Spiegels mit einem Expressionsspiegel, der in Abwesenheit der Verbindung erhalten wird, und
c) das Auswählen von Verbindungen, die die Expression von TNFα oder die Expression des Reporterproteins im Vergleich zu dem Expressionsspiegel, der in Abwesenheit der Verbindung erhalten wird, verstärken.

3. Verwendung einer cDNA oder einer cRNA, die zu der TTP-mRNA komplementär ist, wobei die cDNA oder cRNA an die TTP-mRNA bindet und deren Translation hemmt, oder eines Anti-TTP-Antikörpers, der spezifisch an TTP bindet und die Bindung von TTP an TNFα-mRNA hemmt, oder von Fragmenten dieser Antikörper, die aus der Gruppe ausgewählt werden, bestehend aus F(ab')₂-, F(ab)₂-, Fab'-, Fab-, Fv-Fragmenten, scFv-Proteinen, einwertigen 3,5S-Fab'-Fragmenten und CDR-Peptiden, zur Herstellung eines Arzneimittels zur Behandlung von Herzkrankheiten und/oder Herzverletzungen, ausgewählt aus der Gruppe, bestehend aus hämodynamischer Überlastung, Myokard-Reperfusionsverletzung, hypertropher Kardiomyopathie, kongestiver Herzinsuffizienz im Endstadium, Myokardinfarkt und instabiler Angina.

4. Arzneimittel zur Behandlung von Herzkrankheiten und/oder Herzverletzungen, ausgewählt aus der Gruppe, bestehend aus hämodynamischer Überlastung, Myokard-Reperfusionsverletzung, hypertropher Kardiomyopathie, kongestiver Herzinsuffizienz im Endstadium, Myokardinfarkt und instabiler Angina, enthaltend mindestens eine cDNA oder eine cRNA, die zu der TTP-mRNA komplementär ist, wobei die cDNA oder cRNA an die TTP-mRNA bindet und deren Translation hemmt, oder einen Anti-TTP-Antikörper, der spezifisch an TTP bindet und die Bindung von TTP an TNFα-mRNA hemmt, oder Fragmente dieser Antikörper, die aus der Gruppe ausgewählt werden, bestehend aus F(ab')₂-, F(ab)₂-, Fab'-, Fab-, Fv-Fragmenten, scFv-Proteinen, einwertigen 3,5S-Fab'-Fragmenten und CDR-Peptiden, sowie gegebenenfalls pharmazeutisch unbedenkliche Trägerstoffe, Verdünnungsmittel oder HIIfsstoffe.

## Revendications

1. Méthode de criblage d'un composé en ce qui concerne son aptitude à améliorer la production du facteur de nécrose tissulaire α (TNFα) par l'inhibition et/ou l'inversion de la fixation de tristétraproline (TTP) à l'ARNm de TNFα, comprenant :
a) la mise en contact du composé avec un échantillon dépourvu de cellules comprenant de l'ARNm de TNFα ou avec un échantillon dépourvu de cellules comprenant un produit de recombinaison d'expression capable d'exprimer une partie de la région 3' non traduite de l'ARNm de TNFα qui englobe les fragments d'élément riche en AU (ARE) connus comme déstabilisant l'ARNm de TNFα fusionné à un gène rapporteur en présence de TTP dans des conditions telles que la protéine rapporteur puisse être exprimée, et
b) la détermination du taux d'expression de TNFα ou de la protéine rapporteur et la comparaison de ce taux à un taux d'expression obtenu en absence du composé, et
c) la sélection de composés qui améliorent l'expression de la protéine rapporteur par rapport au taux d'expression obtenu en absence du composé.

2. Méthode de criblage d'un composé en ce qui concerne son aptitude à améliorer la production du TNFα par l'inhibition et/ou l'inversion de la fixation de TTP à l'ARNm de TNFα, comprenant :
a) la mise en contact du composé avec une lignée cellulaire exprimant la TTP et le TNFα ou, au lieu du TNFα, une partie de la région 3' non traduite de l'ARNm de TNFα qui englobe les fragments ARE connus comme déstabilisant l'ARNm de TNFα fusionné à un gène rapporteur, et
b) la détermination du taux d'expression de TNFα ou de la protéine rapporteur et la comparaison de ce taux à un taux d'expression obtenu en absence du composé, et
c) la sélection de composés qui améliorent l'expression de TNFα ou l'expression de la protéine rapporteur par rapport au taux d'expression obtenu en absence du composé.

3. Utilisation d'un ADNc ou d'un ARNc complémentaire de l'ARNm de TTP, ledit ADNc ou ARNc se fixant à et inhibant la traduction de l'ARNm de TTP ou d'un anticorps anti-TTP se fixant spécifiquement à la TTP et inhibant la fixation de TTP à l'ARNm de TNFα ou de fragments desdits anticorps choisis dans le groupe constitué par les fragments F(ab')₂, F(ab)₂, Fab', Fab, Fv, les protéines scFv, les fragments monovalents 3,5S Fab' et les peptides CDR, pour l'élaboration d'un médicament destiné au traitement de maladies cardiaques et/ou de lésions cardiaques choisies dans le groupe constitué par la surcharge hémodynamique, les lésions de reperfusion myocardique, la cardiomyopathie hypertrophique, l'insuffisance cardiaque congestive terminale, l'infarctus du myocarde et l'angine instable.

4. Médicament destiné au traitement de maladies cardiaques et/ou de lésions cardiaques choisies dans le groupe constitué par la surcharge hémodynamique, les lésions de reperfusion myocardique, la cardiomyopathie hypertrophique, l'insuffisance cardiaque congestive terminale, l'infarctus du myocarde et l'angine instable, comprenant au moins un ADNc ou un ARNc complémentaire de l'ARNm de TTP, ledit ADNc ou ARNc se fixant à et inhibant la traduction de l'ARNm de TTP ou un anticorps anti-TTP se fixant spécifiquement à la TTP et inhibant la fixation de TTP à l'ARNm de TNFα ou des fragments desdits anticorps choisis dans le groupe constitué par les fragments F(ab')₂, F(ab)₂, Fab', Fab, Fv, les protéines scFv, les fragments monovalents 3,5S Fab' et les peptides CDR, et éventuellement un véhicule, diluant ou excipient pharmaceutiquement acceptable.
